Europäisches Patentamt

(19) European Patent Office

Office européen des brevets

(11) Numéro de publication: **0 086 680**
**B1**

(12) **FASCICULE DE BREVET EUROPEEN**

(45) Date de publication du fascicule du brevet:
18.12.85

(21) Numéro de dépôt: 83400079.6

(22) Date de dépôt: 13.01.83

(51) Int. Cl.⁴: **C 07 D 307/62**, C 07 C 101/22,
C 07 C 101/30, C 07 C 149/43,
A 61 K 31/375

(54) Nouvelles compositons à effet antiasthénique puissant à base de sels doubles de l'acide ascorbique et d'un diacide amine.

(30) Priorité: 15.02.82 FR 8202407

(43) Date de publication de la demande:
24.08.83 Bulletin 83/34

(45) Mention de la délivrance du brevet:
18.12.85 Bulletin 85/51

(84) Etats contractants désignés:
BE CH DE GB IT LI LU NL SE

(56) Documents cités:
FR - A - 1 182 874
FR - A - 1 316 208
FR - A - 1 510 731
FR - M - 6 859
GB - A - 752 547

(73) Titulaire: **LABORATOIRES MAYOLY SPINDLER,
16 Avenue des Châteaupleds, F-92502 Ruell-Malmaison
(FR)**
Titulaire: **Vernin, née Gruson, Christiane, 343 Avenue des
Courtillerales, F-77350 Le Mee s/Seine (FR)**
Titulaire: **Vernin, Jean-Gilles, 14 Place de la Porte des
Ternes, F-75017 Paris (FR)**
Titulaire: **Pascalet, née Vernin, Patricia, Résidence La
Hunière Rue Gensollen, Bât. C., F-83000 Toulon (FR)**

(72) Inventeur: **Vernin, Jean-Marie, Décédé (FR)**

(74) Mandataire: **Ores, Irène et al, CABINET ORES 6, Avenue
de Messine, F-75008 Paris (FR)**

Il est rappelé que: Dans un délai de neuf mois à compter de la date de publication de la mention de la délivrance du brevet euro-péen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention est relative à de nouvelles compositions à effet antiasthénique puissant et à leurs procédés de préparation.

On connaît de nombreux médicaments à effet antiasthénique et détoxicant utilisés avec plus ou moins de succès dans le cas de fatigues (et notamment fatigues intellectuelles et psychiques), dans le cas de surmenage, de convalescence et de sénescence (cf. notamment les Brevets GB-A-752 547, FR-A-1 510 731 et FR-A-1 182 874).

Après des années d'étude aussi bien in vitro qu'in vivo dans de nombreuses cliniques, et notamment en suivant l'action antiasthénique du médicament décrit dans le Brevet Spécial de Médicament n° 6859, à savoir l'action antiasthénique du sel double de l'acide ascorbique et de l'acide aspartique avec un métal divalent, la Demanderesse s'est fixé pour but de pourvoir à une nouvelle composition qui répond mieux aux nécessités de la pratique que les médicaments visant au même but antérieurement connus, et notamment pourvoir à une nouvelle composition qui traite non seulement les asthénies de toutes natures, aussi bien physiques que psychiques, mais également les dénutritions, les convalescences, le catabolisme sénile, l'asthénie sexuelle, le dysfonctionnement métabolique, l'anxiété, l'insomnie et la dépression.

La présente invention a pour objet une nouvelle composition à effet antiasthénique et détoxicant, caractérisée en ce qu'elle est constituée d'une pluralité de sels doubles de l'acide ascorbique et d'un diacide aminé d'un métal divalent pharmaceutiquement compatible.

Conformément à l'invention, les acides aminés sont pris dans le groupe qui comprend la cystine, l'acide aspartique, l'acide glutamique, l'acide hydroxy-3 glutamique et la lanthionine.

Selon un mode de réalisation particulièrement avantageux, la composition conforme à la présente invention comprend comme acides aminés:

- de  0 à 30% de la cystine;
- de 10 à 40% de l'acide aspartique;
- de 15 à 40% de l'acide glutamique;
- de 10 à 25% de l'acide hydroxy-3 glutamique; et
- de  0 à 15% de la lanthionine.

Selon un autre mode de réalisation particulièrement avantageux de l'objet de l'invention, le métal divalent est constitué par le magnésium et la composition comprend (poids/poids):

- 33% de l'ascorbo-aspartate de magnésium;
- 30% de l'ascorbo-glutamate de magnésium;
- 18% de l'ascorbo-hydroxy-3 glutamate de magnésium;
- 15% de l'ascorbo-cystinate de magnésium; et
- 4%  de l'ascorbo-lanthioninate de magnésium.

Le procédé de préparation de ces différents sels doubles est inspiré du procédé décrit dans le Brevet Français n° 1 316 208 qui concerne un sel double ascorbo-aspartique. On opère par exemple ainsi: on fait dissoudre sous atmosphère inerte dans de l'eau distillée de l'acide ascorbique et un diacide aminé.

On ajoute ensuite le carbonate de magnésium et on filtre. Le sel double est précipité par addition d'alcool éthylique.

L'invention pourra être mieux comprise à l'aide du complément de description qui va suivre, qui donne un compte-rendu d'expérimentation pharmacologique destiné à démontrer la remarquable action thérapeutique de nouvelles compositions conformes à la présente invention.

Il doit être bien entendu, toutefois, que ce compte-rendu est donné uniquement à titre d'illustration de l'objet de l'invention et n'a aucun caractère limitatif.

*COMPTE RENDU PHARMACOLOGIQUE*

1) *Toxicité:*

On a testé aussi bien la toxicité aiguë que la toxicité chronique: on a constaté une tolérance parfaite, celle habituellement constatée pour les acides aminés et la vitamine C.

2) *Action sur l'épuisement des animaux soumis à l'épreuve de nage forcée:*

Cette action, destinée à démontrer l'activité antifatigue de la nouvelle composition, est mise en évidence à l'aide de l'épreuve de nage en utilisant la nouvelle composition conforme à la présente invention.

Cette expérimentation a été réalisée sur des souris de la souche Swiss Albinos de deux sexes d'un poids moyen de 20 g, lestées à l'aide de charges calculées en fonction de leur poids corporel.

Ces souris ont été partagées en trois lots:

a) Témoin (10 souris);
b) Souris auxquelles on a administré 1 mg d'ascorbo-aspartate de magnésium (brevet Français BSM 6859) par ml d'eau de boisson pendant 24 heures qui ont précédé l'épreuve de nage (20 souris);
c) Souris auxquelles on a administré 1 mg de produit conforme à la présente invention par ml d'eau de boisson pendant 24 heures qui ont précédé l'épreuve de nage (également 20 souris).

La composition du produit conforme à la présente invention était la suivante:

- Ascorbo-aspartate de magnésium        33%
- Ascorbo-glutamate de magnésium        30%
- Ascorbo-hydroxy-3 glutamate de magnésium 18%
- Ascorbo-cystinate de magnésium        15%
- Ascorbo-lanthioninate de magnésium      4%

L'activité remarquable du produit conforme à la présente invention a été mise en évidence par la comparaison du temps de nage des animaux du troisième lot. Les résultats sont résumés dans le TABLEAU I ci-après:

### TABLEAU I

| LOT | TEMPS DE NAGE |
|---|---|
| Témoin | Moyenne sur 10 souris 6 minutes 5 secondes |
| Souris traitées uniquement à l'ascorbo-aspartate de magnésium | Moyenne sur 20 souris 9 minutes 6 secondes |

TABLEAU I (suite)

| LOT | TEMPS DE NAGE |
|---|---|
| Souris traitées au produit conforme à la présente invention | Moyenne sur 20 souris 14 minutes 40 secondes |

La restauration des forces des animaux traités par le produit conforme à la présente invention a été également plus rapide que celle des deux autres groupes.

Il ressort des propriétés pharmacologiques dont il vient d'être rendu compte, que les nouvelles compositions conformes à la présente invention doivent être considérées comme antiasthéniques de choix pour les traitements préventifs et curatifs des états de fatigue physique ou intellectuelle et dans les états de fatigue nerveuse, tels, notamment que le surmenage scolaire ou professionnel, les convalescences des maladies infectieuses, les asthénies de diverses origines et, en particulier, d'origine post-grippale.

Les nouvelles compositions conformes à l'invention peuvent être administrées soit par la voie orale (de préférence), soit par voie rectale. En particulier, elles peuvent être présentées en ampoules buvables dosées à 0,80 mg de constituant actif.

## Revendications

1. Composition à effet antiasthénique et détoxicant, caractérisé en ce qu'elle est constituée d'une pluralité de sels doubles de l'acide ascorbique et d'un diacide aminé, pris dans le groupe qui comprend la cystine, l'acide aspartique, l'acide glutamique, l'acide hydroxy-3 glutamique et la lanthionine, avec un métal divalent pharmaceutiquement compatible.

2. Composition selon la revendication 1, caractérisée en ce qu'elle comprend comme acides aminés:

- de  0 à 30% de la cystine,
- de 10 à 40% de l'acide aspartique,
- de 15 à 40% de l'acide glutamique,
- de 10 à 25% de l'acide hydroxy-3 glutamique, et
- de  0 à 15% de la lanthionine.

3. Composition selon l'une quelconque des revendications 1 et 2, caractérisée en ce que le métal divalent est constitué par le magnésium.

4. Composition selon l'une quelconque des revendications 1 à 3, caractérisée en ce qu'elle comprend (poids/poids):

- 33% de l'ascorbo-aspartate de magnésium,
- 30% de l'ascorbo-glutamate de magnésium,
- 18% de l'ascorbo-hydroxy-3 glutamate de magnésium,
- 15% de l'ascorbo-cystinate de magnésium,
- 4%  de l'ascorbo-lanthioninate de magnésium.

## Patentansprüche

1. Komposition mit anti-entkräftigendem und detoxifizierendem Effekt, dadurch gekennzeichnet, dass sie dargestellt wird durch eine Vielzahl von Doppelsalzen von Ascorbinsäure und von Amino-Dicarbonsäure, genommen aus der Gruppe, die das Cystin, die Asparaginsäure, die Glutaminsäure, die 3-Hydroxy-Glutaminsäure und das Lanthionin umfasst, mit einem zweiwertigen pharmazeutisch kompatiblen Metall.

2. Komposition gemäss Anspruch 1, dadurch gekennzeichnet, dass sie als Aminosäuren

- von  0 bis 30% Cystin,
- von 10 bis 40% Asparaginsäure,
- von 15 bis 40% Glutaminsäure,
- von 10 bis 25% 3-Hydroxy-Glutaminsäure, und
- von  0 bis 15% Lanthionin
umfasst.

3. Komposition gemäss einem der Ansprüche 1 und 2, dadurch gekennzeichnet, dass das zweiwertige Metall Magnesium ist.

4. Komposition gemäss einem der Ansprüche 1 bis 3, dadurch gekennzeichnet, dass sie (Gewicht/Gewicht)

- 33% Magnesium-Ascorbo-Aspartat,
- 30% Magnesium-Ascorbo-Glutamat,
- 18% Magnesium-Ascorbo-3-Hydroxy-Glutamat,
- 15% Magnesium-Ascorbo-Cystinat,
- 4% Magnesium-Ascorbo-Lanthioninat
umfasst.

## Claims

1. A composition having an antiasthenic and detoxicating effect, characterized in that it consists of several double salts of ascorbic acid and of an amino diacid taken from the group comprising cystine, aspartic acid, glutamic acid, 3-hydroxyglutamic acid and lanthionine, with a pharmaceutically compatible divalent metal.

2. A composition according to claim 1, characterized in that it comprises the following as amino acids:

- from  0 to 30% of cystine,
- from 10 to 40% of aspartic acid,
- from 15 to 40% of glutamic acid,
- from 10 to 25% of 3-hydroxyyglutamic acid and
- from  0 to 15% of lanthionine.

3. A composition according to either one of claims 1 and 2, characterized in that the divalent metal consists of magnesium.

4. A composition according to any one of claims 1 to 3, characterized in that it comprises (weight/weight):

- 33% of magnesium-ascorbo-aspartate,
- 30% of magnesium-ascorbo-glutamate,
- 18% of magnesium-ascorbo-3-hydroxyglutamate,
- 15% of magnesium-ascorbo-cystinate and
- 4%  of magnesium-ascorbo-lanthioninate.